(19) Europäisches Patentamt
European Patent Office
Office européen des brevets

(11) Publication number: 0 104 646
A1

# (12) EUROPEAN PATENT APPLICATION

(21) Application number: 83109601.1

(22) Date of filing: 27.09.83

(51) Int. Cl.³: C 07 D 307/32

(30) Priority: 28.09.82 JP 170206/82

(43) Date of publication of application: 04.04.84 Bulletin 84/14

(84) Designated Contracting States: AT BE CH DE FR GB IT LI NL

(71) Applicant: Takeda Chemical Industries, Ltd.
27, Doshomachi 2-chome Higashi-ku
Osaka-shi Osaka, 541(JP)

(72) Inventor: Morimoto, Manzaemon
1-39, Midoridai 5-chome
Kawanishi Hyogo 666-01(JP)

(72) Inventor: Sugimoto, Keiichi
11-10, Daiwahigashi 1-chome
Kawanishi Hyogo 666-01(JP)

(74) Representative: von Kreisler, Alek, Dipl.-Chem. et al,
Deichmannhaus am Hauptbahnhof
D-5000 Köln 1(DE)

(54) **Method of preparing L-gulono-gamma-lactone.**

(57) A method for preparing L-gulono-γ-lactone which is a very useful compound employed in a broad range as starting materials of, among others, pharmaceuticals and agricultural chemicals, which is characterized by subjecting L-ascorbic acid to catalytic reduction under elevated pressure in a solvent in the presence of a ruthenium catalyst.

EP 0 104 646 A1

0104646

# Method of Preparing L-Gulono-γ-lactone

This invention relates to a method of preparing L-gulono-γ-lactone which is a very useful compound employed in a broad range as starting materials of, among others, pharmaceuticals and agricultural chemicals.

More specifically, this invention relates to an industrially advantageous method of preparing L-gulono-γ-lactone with a good yield and a high selectivity coefficient by subjecting L-ascorbic acid to catalytic reduction in a solvent under elevated pressure in the presence of ruthenium catalyst. As advantageous reduction of L-ascorbic acid, there has been known a method of subjecting it to catalytic reduction using as the catalyst a 10% palladium-carbon, more concretely, conducting the reduction under hydrogen pressure of 50 Psi (pound square inch) at 50°C for 24 hours using the above-mentioned catalyst in an amount corresponding to 10% (w/w = weight per weight) relative to the L-ascorbic acid to be reduced [The Journal of Organic Chemistry (1981), 46, pp. 2976-2977].

However, repeated follow-ups of the above known method by the present inventors confirmed that the conversion is low, the yield does not exceed 65-70% and reproduction of quantitative yield is hardly expected because of relatively large amount of by-products.

This is surmised to be due to the following: As have been well known, catalytic reduction of a compound having enol-lactone ring is accompanied by hydrogenolysis to form desoxy acid, and hydrogenation of enol and rupture of lactone ring are competitive reactions, and thus the method using the above-mentioned palladium-type catalyst cannot necessarily be regarded as giving a satisfactory yield percentage of the desired L-gulono-γ-lactone, i.e. a satisfactory selectivity coefficient of saturated lactone,

and besides, the amount of by-products formed by rupture of the lactone ring is relatively large.

The present inventors conducted diligent research work on finding out a catalyst by which reduction of L-ascorbic acid can be conducted with an industrial advantage. As a result, it was unexpectedly found that the catalytic reduction of L-ascorbic acid under elevated pressure in a solvent in the presence of a ruthenium catalyst renders the reaction to proceed in a short period of time at a low temperature in the presence of a very small amount of the catalyst to give L-gulono-$\gamma$-lactone of a high purity in a good yield, even in its industrial scale production e.g. in terms of Kg. level. Based on this finding, the present invention was accomplished.

This invention thus relates to a method of preparing L-gulono-$\gamma$-lactone, which is characterized by subjecting L-ascorbic acid to catalytic reduction under elevated pressure in a solvent in the presence of ruthenium catalyst.

As the ruthenium catalyst employable in the method of this invention, there may be mentioned ruthenium itself, or ruthenium oxide such as ruthenium dioxide, ruthenium tetroxide, or ruthenium or ruthenium oxide as adsorbed by a known method on an adsorbing agent e.g. diatomaceous earth, silica or activated charcoal, especially 1-10% (w/w), preferably 2-5% (w/w) ruthenium-activated charcoal. The used amount of the catalyst is in the range of 2-10% (w/w), especially preferably 3-5% (w/w) relative to L-ascorbic acid. The ruthenium catalyst can be repeatedly used without further re-activation.

As the solvent, there may be exemplified water, alcohols such as ethyl alcohol or methyl alcohol or a mixture thereof, and, among them, water is most preferable in view of high solubility of the starting material in water. The volume of solvent used is not limited so long as the reaction proceeds, but, in general, it ranges from 3 mℓ to 30 mℓ, preferably 5-10 mℓ, relative to 1 g of L-ascorbic acid. The initial

pressure (i.e., the pressure when the reduction starts) is usually 5-300 $Kg/cm^2$, preferably 10-100 $Kg/cm^2$. The pressure is applied usually with hydrogen gas, or with a mixture of an inert gas such as helium, argon carbon dioxide or nitrogen with hydrogen. The reaction temperature ranges from 0°C to 60°C, preferably 10-45°C. More specifically, the reduction is conducted under 30 $Kg/cm^2$ - 100 $Kg/cm^2$ and at a temperature of 15-40°C to give a preferable result. The reaction time varies, among others, with kinds and amounts of the catalysts used, the reaction pressure and temperature employed, types of the autoclave or rotation velocity of the stirrer, and the reaction usually completes within 7 hours. For example, stirring is conducted preferably for about 30 minutes to 5-6 hours at 60-150 r.p.m. (rotation per minute). The volume of hydrogen supplied to the reduction reaction is at least 1 mole (preferably 1 to 2 moles) relative to 1 mole of L-ascorbic acid. Other reaction conditions are analogous to those employed in common reduction processes in a liquid phase under high pressure. Thus-prepared end-product can be separated by conventional means, for example, taking the reaction mixture out of the autoclave, separating the catalyst from the reaction mixture by filtration and evaporating off the solvent from the filtrate under reduced pressure. Further, upon necessity, thus separated end-product is suspended in an alcohol (e.g. methanol or ethanol) or an aqueous alcohol and the suspension is warmed, followed by cooling to cause crystallization to yield highly pure crystals of L-gulono-γ-lactone.

As explained in the foregoing, according to the method of this invention of preparing L-gulono-γ-lactone by reducing L-ascorbic acid in the presence of a ruthenium catalyst, a relatively small amount of the catalyst is sufficient, a high conversion rate can be reached at a low temperature for a very short period of time (in other words, absorption rate of $H_2$ is high), and a highly pure end-product can be prepared with a high yield by a simple after-treatment, thus the method of this invention for preparing L-gulono-

γ-lactone is very advantageous as an industrial-scale production, i.e. in terms of Kg. level.

Fig. 1 shows that, in the cases ① (Example 1) and ② (Example 2) of this invention employing Ru-C at 24-32°C and 40°C respectively, absorption rates of $H_2$ are remarkably higher (conversion rate is higher) than those in cases ③ (Reference Example 1) and ④ (Reference Exmaple 2) of a known method employing Pd-C at 50°C. In Fig. 2, ⑤, ⑥, ⑦ and ⑧ respectively show the absorption rates of $H_2$ in Examples 5 and 6, and Reference Examples 3 and 4. It is clear that the conversion rates of ⑤ and ⑥ in this invention are higher than those of ⑦ and ⑧ in the known method.

Thus prepared L-gulono-γ-lactone has a wide range of uses as intermediates or materials for chemical synthesis and can be used as starting materials for medicines, agricultural chemicals, etc. For instance, L-gulono-γ-lactone is allowed to react with P-phenetidine or P-anisidine to give N-(P-ethoxyphenyl)-L-gulonic amide or N-(P-methoxyphenyl)-L-gulonic amide, which are antipyretic and analgesic agents (c.f. Japanese Published examined patent application No. 21240/1964), or L-gulonic acid, prepared by allowing L-gulono-γ-lactone to pass through strongly basic ion exchange resin (e.g. Amberlite IRA-410 ($OH^-$), etc.) and then neutralizing it, is allowed to react with 3-formyl-7-(2-thienylacetamido)-3-cephem-4-carboxy-hemi-acetal lactone to give an antibacterial substance representable by the formula;

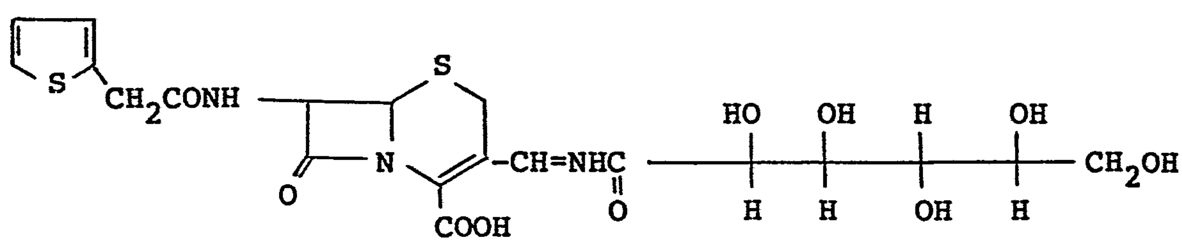

(Japanese Published unexamined patent application No. 131982/1975), and, besides, L-gulono-γ-lactone can be used as materials for synthesizing a variety of compounds. (Advances in Carbohydrate Chemistry and Biochemistry, 1981, Vol. 38, pp. 287-321)

By means of the following working examples, the method of this invention will be explained in detail, but they are not intended to limit the scope of this invention.

Example 1

A one-litre capacity autoclave equipped with a vertical type magnetic stirrer was charged with 80 g of L-ascorbic acid, 580 mℓ of pure water and, as a catalyst, 4 g of 5% ruthenium-carbon. Hydrogen gas was introduced into the autoclave until the pressure reached 100 $Kg/cm^2$, and the mixture was stirred at 24-32°C. In two hours, the reaction ceased while theoretical amount of hydrogen was absorbed into the reaction mixture. The reaction product was taken out of the autoclave, from which the catalyst was removed by filtration and then water was evaporated off. To the residue was added 80 mℓ of methyl alcohol to make a suspension. The suspension was heated up to 60°C, then cooled to 5°C. The resulting crystals were collected by filtration, and dried at 40°C under reduced pressure to give 70.4 g (yield 87%) of white crystalline L-gulono-γ-lactone. Purity of this product was determined by means of gas-chromatography, which was found to be 98%.

$[\alpha]_D^{25}$=+56.4° (c=1.0, $H_2O$)
melting point (m.p.) 182-184°C

Example 2

The same reaction as in Example 1 was conducted, excepting using 8 g of 5% ruthenium and conducting the reaction at 40°C. In 30 minutes, the reaction ceased while absorbing theoretical volume of hydrogen. The reaction product was treated in a manner similar to that in Example 1 to afford 74.4 g (yield 92%) of white crystalline L-gulono-γ-lactone, the purity of which was determined by means of gas-chromatography as 99%.

$[\alpha]_D^{25}$=+56.5° (c=1.0, $H_2O$)
melting point (m.p.) 183-185°C

Example 3

An autoclave of two-litre capacity, equipped with an induction-type rotary stirrer, was charged with 120 g of L-ascorbic acid, 800 m*l* of pure water and 12 g of 5% ruthenium-carbon. The reaction was conducted at 30-31°C under hydrogen pressure of 10 Kg/cm, and the reaction ceased in 5.5 hours. Determination of the reaction solution by means of gas-chromatography revealed that the yield of L-gulono-γ-lactone was 94%.

Example 4

An autoclave of 20-litre capacity, equipped with a horizontal stirrer, was charged with 1.2 Kg of L-ascorbic acid, 8 *l* of pure water and 60 g of 5% ruthenium-carbon. The reaction was conducted at a temperature of 28-31°C under 100 $Kg/cm^2$ hydrogen pressure. In three hours, the theoretical volume of hydrogen was absorbed and the reaction ceased. The reaction mixture was processed in a manner analogous to that of Example 1 to give 1.09 kg (yield 90%) of L-gulono-γ-lactone. Purity of this product was determined by means of gas-chromatography as 98.5%.

$[\alpha]_D^{25}$=+56.4° (c=1.0, $H_2O$)
melting point (m.p.) 183-185°C

Example 5

A one-litre capacity autoclave equipped with a vertical type magnetic stirrer was charged with 80 g of L-ascorbic acid, 580 m*l* of pure water and 8 g of 10% ruthenium-carbon. Hydrogen gas was introduced until the pressure reached 5 $kg/cm^2$, and the reaction was conducted at 50°C. In six hours, the reaction ceased while theoretical amount of hydrogen was absorbed into the reaction mixture. Analysis of the composition of the reaction product by means of gas-chromatography revealed as follows:

| | |
|---|---|
| L-gulono-γ-lactone | 84.3% |
| unreacted L-ascorbic acid | 0.3% |
| unknown substances | 15.4% |

## Example 6

Example 5 was repeated, excepting that 4 g of 5% ruthenium-carbon was employed and the reaction was conducted at 31-32°C under 100 $kg/cm^2$ of hydrogen pressure. In one hour and forty minutes, the reaction was ceased while theoretical amount of hydrogen was absorbed into the reaction mixture. Analysis of the composition of the reaction product by means of gas-chromalography revealed as follows:

| | |
|---|---|
| L-gulono-γ-lactone | 88.9% |
| unreacted L-ascorbic acid | 0 |
| unknown substances | 11.1% |

## Examples 7-10

Example 6 was repeated, excepting that 5% ruthenium-carbon once used in Example 6 was separated by filtration and washed with water was repeatedly used. The results were shown by the following table.

| Example No. | Reaction | | Analysis by means of gas-chromatography | | |
|---|---|---|---|---|---|
| | Hour | Amount of $H_2$ absorbed | L-gulono-γ-lactone | Unreacted L-ascorbic acid | Unknown substances |
| | Hour min. | | | | |
| 7 | 1 : 30 | Theoretical | 91.3% | 0% | 8.7% |
| 8 | 1 : 10 | " | 90.4% | 0 | 9.6 |
| 9 | 1 : 00 | " | 95.2% | 0 | 4.8 |
| 10 | 1 : 05 | " | 95.5% | 0 | 4.5 |

Reference Example 1

An autoclave of the same type as employed in Example 1 is charged with 80 g of L-ascorbic acid, 580 mℓ of pure water and 16 g of 5% palladium-carbon. The reaction was conducted at 50°C under 4 $Kg/cm^2$ hydrogen pressure. In eight hours and twenty minutes, the theoretical volume of hydrogen was absorbed and the reaction ceased. The reaction product was processed in a manner similar to that in Example 1 to give 54.6 g (yield: 67.5%) of white crystalline L-gulono-γ-lactone, the purity of which was 97.8% according to gas-chromatographic determination.

$[\alpha]_D^{25}$=+57.1° (c=1.0, $H_2O$)

melting point (m.p.) 182.5-184.5°C

Reference Example 2

An autoclave of a similar type to that employed in Example 4 was charged with 1.2 Kg of L-ascorbic acid, 8ℓ of pure water and 240 g of 5% palladium-carbon. The reaction was allowed to proceed at 50°C under 4 $Kg/cm^2$ of hydrogen pressure, but absorption of hydrogen took long time, and, in 19 hours and 50 minutes, 87.4% of hydrogen relative to the theoretical volume was absorbed, but no absorption was observed thereafter. The reaction was suspended. The reaction mixture was processed in a manner similar to Example 4 to give 838.5 g (69.1%) of L-gulono-γ-lactone, the purity of which was 96.7% according to gas-chromatographic determination.

$[\alpha]_D^{25}$=+56.3° (c=1.0, $H_2O$)

melting point (m.p.) 181.5-184.5°C

Reference Example 3

Example 5 was repeated excepting that 8 g of 10% palladium-carbon was used. After the lapse of tweleve hours and twenty minutes, 78.2% of the theoretical amount of hydrogen gas was absorbed and no more absorption was observed thereafter, hence the reaction was suspended. Analysis of the composition of the reaction solution by means of gas-chromatography gave the following results.

| | |
|---|---|
| L-gulono-γ-lactone | 66.5% |
| unreacted L-ascorbic acid | 14.3% |
| unknown substances | 19.2% |

Reference Example 4

Example 6 was repeated excepting that 4 g of 5% palladium-carbon was used. As the absorption of hydrogen gas did not proceed well, the reaction temperature was raised up to 50°C, but the absorption rate was extremely slow. The reaction temperature was then raised further up to 80°C. In five hours and twenty-five minutes, an amount of hydrogen gas corresponding to 73.5% relative to the theoretical was absorbed. Thereafter, no absorption of hydrogen gas was observed, and the reaction was suspended. Analysis of the composition of the reaction solution by means of gas-chromatography gave the following results.

| | |
|---|---|
| L-gulono-γ-lactone | 50.4% |
| unreacted L-ascorbic acid | 26.3% |
| unknown substances | 23.3% |

What we claim is:

1. A method for preparing L-gulono-γ-lactone which comprises subjecting L-ascorbic acid to catalytic reduction under elevated pressure in a solvent in the presence of a ruthenium catalyst.

2. A method according to Claim 1, wherein the ruthenium catalyst is (1) ruthenium, (2) a ruthenium oxide or (3) ruthenium or ruthenium oxide adsorbed on an adsorbing agent.

3. A method according to Claim 2, wherein the ruthenium adsorbed on an adsorbing agent is 1-10% (w/w) ruthenium-carbon.

4. A method according to Claim 1, wherein an amount of the catalyst is in the range of 2-10% (w/w) relative to L-ascorbic acid.

5. A method according to Claim 1, wherein the solvent is water, an alcohol or a mixture thereof.

6. A method according to Claim 1, wherein the solvent is water.

7. A method according to Claim 1, wherein an amount of the solvent is in the range of 3-30 mℓ relative to 1 g of L-ascorbic acid.

8. A method according to Claim 1, wherein the initial pressure is 5-300 $Kg/cm^2$.

9. A method according to Claim 1, wherein the pressure is applied with hydrogen gas or with a mixture of an inert gas with hydrogen.

10. A method according to Claim 1, wherein the volume of hydrogen supplied to the reduction reaction is at least 1 mole relative to 1 mole of L-ascorbic acid.

1/2

Fig. 1

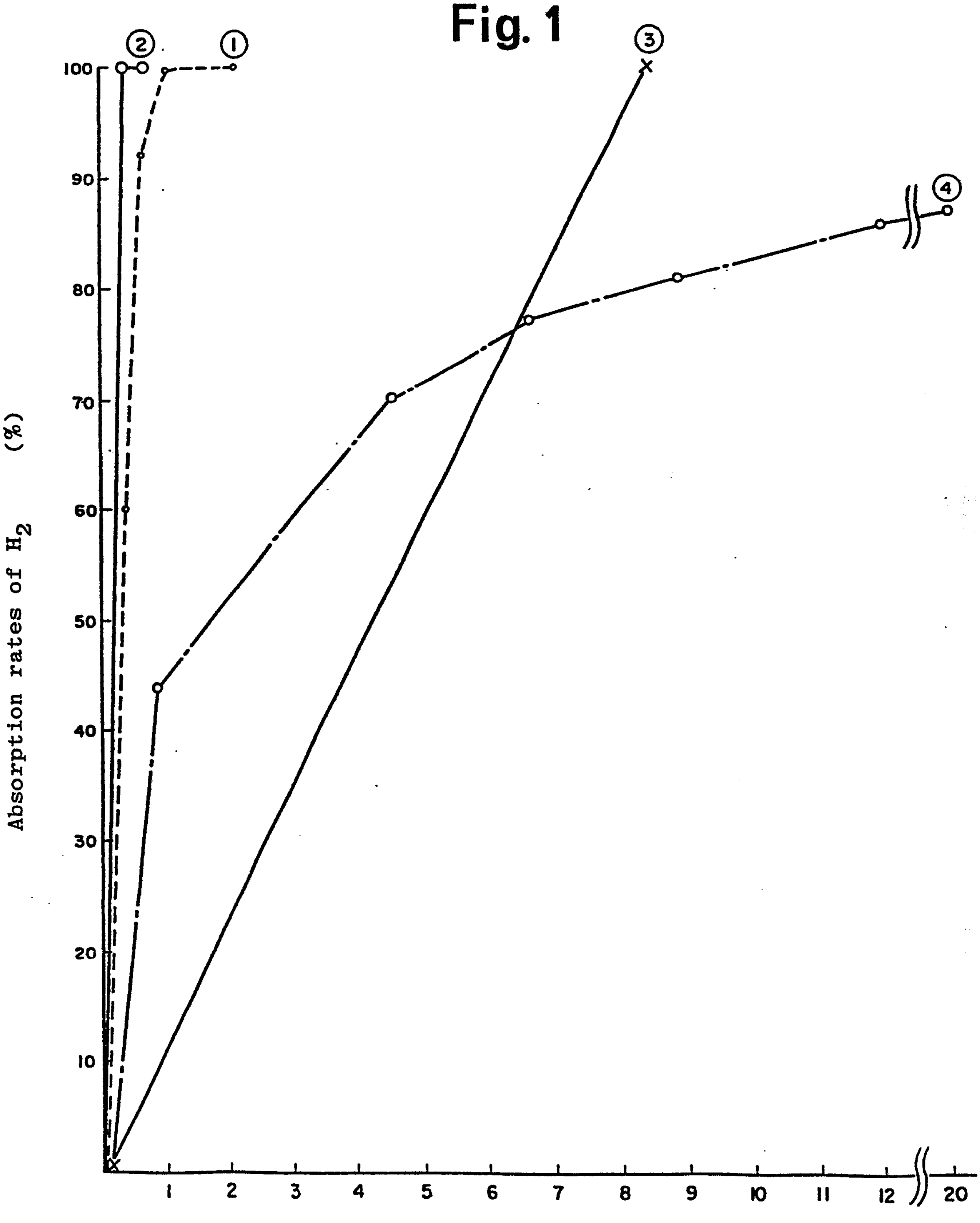

(2)
(1)
(3)
(4)
100
90
80
70
60
50
40
30
20
10
Absorption rates of $H_2$ (%)
1
2
3
4
5
6
7
8
9
10
11
12
20
Hours

# Fig. 2

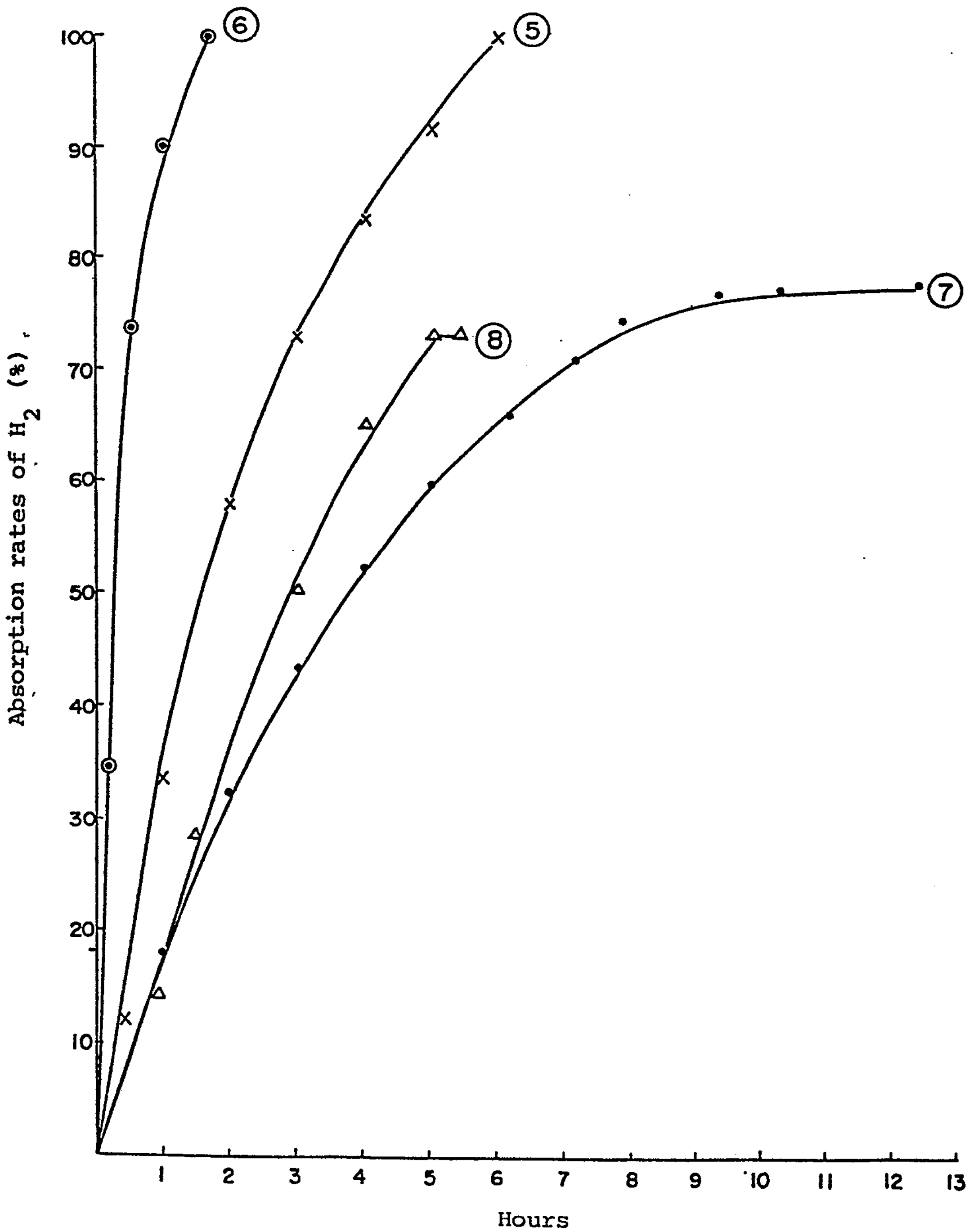
100
90
80
70
60
50
40
30
20
10
Absorption rates of $H_2$ (%)
6
5
8
7
1
2
3
4
5
6
7
8
9
10
11
12
13
Hours

European Patent Office

# EUROPEAN SEARCH REPORT

Application number

EP 83 10 9601

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-4 326 072 (ICI AMERICA) * Whole document * | 1-10 | C 07 D 307/32 |
| D,Y | JOURNAL OF ORGANIC CHEMISTRY, vol. 46, no. 14, 3rd July 1981, pages 2976-2977, American Chemical Society, Columbus, Ohio, US G.C. ANDREWS et al.: "Stereoselective, catalytic reduction of L-ascorbic acid: A convenient synthesis of L-gulono-1,4-lactone" * Whole article * | 1-10 | |
| | | | TECHNICAL FIELDS SEARCHED (Int. Cl. 3) |
| | | | C 07 D 307/00 |

The present search report has been drawn up for all claims

| Place of search | Date of completion of the search | Examiner |
|---|---|---|
| THE HAGUE | 09-12-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82